# EUROPEAN PATENT SPECIFICATION

(11) **EP 1 033 326 B1**
(45) Date of publication and mention of the grant of the patent: **13.10.2004**
(21) Application number: 00301640.9
(22) Date of filing: 01.03.2000
(51) Int. Cl.: B65D 85/00, B65B 55/08, A61L 2/10, A61L 12/06, B32B 27/08

(54) **Package for medical device**
Verpackung für einen medizinischen Behelf
Emballage pour un dispositif médical

(30) Priority: 01.03.1999 US 259795
(43) Date of publication of application: 06.09.2000
(60) Divisional application: 04077111.5
(73) Proprietor: Johnson & Johnson Vision Care, Inc., Jacksonville, FL 32256 (US)
(72) Inventor: Muggli, Oliver Y., Louisville, Kentucky 40245 (US); Peck, James Malcolm, Jacksonville, Florida 32224 (US)
(74) Representative: Mercer, Christopher Paul

(56) References cited:
- EP-A- 0 580 176
- EP-A- 0 765 741
- US-A- 5 618 492
- US-A- 5 786 598
- DATABASE WPI Section Ch, Week 198607 Derwent Publications Ltd., London, GB; Class A92, AN 1986-045815 XP002901024 & JP 61 000175 A (OKURA IND CO LTD), 6 January 1986 (1986-01-06)

## Description

### FIELD OF THE INVENTION

This invention relates broadly to a package for a medical device. More specifically, this invention relates to a package for a medical device which is designed for a UV radiation sterilization method.

### DESCRIPTION OF THE RELATED ART

Medical device sterilization processes, and in particular commercial contact lens manufacturing sterilization processes, typically involve some form of temperature and/or pressure-based sterilization techniques. For example, a hydrophilic contact lens is typically first formed by injecting a monomer mixture into a mold. The monomer mixture is then polymerized (i.e. the lenses are cured). After other optional processing steps, such as quality inspections, the lens is placed into a container with a solution and the container is sealed. The packaged lens is sterilized by placing the container into an autoclave at an elevated temperature and pressure for an extended period of time, usually at least 15 minutes, typically 30 minutes. Although this commercial process produces thoroughly sterilized contact lenses, the batch-wise autoclave sterilization step is time consuming and costly.

European Patent Publication No. 0 222 309 A1 discloses a process using ozone in which packaging material is disinfected in a manufacturing setting. The process involves feeding an oxygen stream into an ozonating chamber, generating ozone from oxygen in the ozonating chamber, placing packaging containers in a sanitizing chamber, feeding the ozone into the sanitizing chamber, and purging the ozone from the sanitizing chamber with sterile air. The process requires that the ozone contact the packaging material for a predetermined time, followed by the sterile air purge step. The process is offered as an alternative to heat-steam sterilization, sterilization by application of electromagnetic radiation, or chemical agent sterilization. Various packaging materials were tested.

U.S. Patent No. 5,618,492 discloses a process for producing a sterile contact lens in a sealed container during a continuous production process wherein the contact lens is immersed in an ozone-containing solution within a container during a continuous lens packaging process, and the lens and container are subsequently subjected to ultraviolet radiation primarily to degrade the ozone. This process sterilizes the contact lens and the container. The materials of the container are not described.

Non-ionizing radiation such as ultraviolet (UV) light is known to damage the DNA of exposed cells. The UV light causes bonds to form thymine dimers which inhibit replication of DNA during cell reproduction. UV light is used for disinfection in hospital rooms, nurseries, operating rooms and cafeterias. UV light is also used to sterilize vaccines, serum, toxins, municipal waste, and drinking waters. The major weakness of the efficacy of UV light as a sterilizer is that for most materials the radiation is not very penetrating, so the microorganisms to be killed must be directly exposed to the radiation.

A number of patents teach the application of UV light to disinfect and/or inactivate microorganisms to either reduce populations of microorganisms or to eliminate them.

US Patent 5,768,853 and WO96/09775 describe the use of a UV light producing apparatus which deactivates microorganisms in food.

US Patent 4,464,336 suggests a method of sterilization by using a flash discharge ultraviolet lamp. The patent teaches that by applying short duration high intensity UV light that microorganisms will be destroyed; however, the conditions for sterilization are not disclosed, nor its application for medical devices.

US Patent 5,786,598 and WO 97/43915 disclose the broad concept that a flash lamp system might be used for deactivating microorganisms in containers. Disclosed containers include IV bags, and a polyolefin container for a contact lens and a preservative fluid. Preservation is the use of physical and/or chemical means to kill or prevent the growth of those microorganisms which, by their growth and/or activities, may cause bio-deterioration of a given material or product. P. Singleton and D. Sainsbury, 1988. Dictionary of Microbiology and Molecular Biology, John Wiley & Sons, New York, NY, pp. 702-703. Although the patent discloses the idea of using a flash lamp system to sterilize contact lenses in a preserved solution in a container, there are no conditions defined to accomplish sterility, nor examples which show that sterility can be accomplished. Further, potentially useful container materials are only suggested.

U.S. Patents 5,034,235 and 4,871,559 disclose the use of intermittent pulses of very intense, very short duration pulses of light to inactivate microorganisms on the surface of food products, and suggests that the method can be used for packages, medical devices, and food products in packages.

EP Publication No. 0 765 741 A1 discloses a lidstock for a contact lens container comprising a clear laminated plastic structure. The lidstock has a label and comprises three layers: two plastic layers and a barrier layer. The printed label will block UV radiation.

There still remains a need for a container for housing a medical device and/or a liquid that would be useful for a UV radiation method of sterilization, that would provide an adequate shelf life during which the container would be impenetrable to microorganisms or vapor, and not subject to attack by the atmosphere.

### SUMMARY OF THE INVENTION

This invention provides a medical device container comprising thermoplastic materials wherein said container is transmissive over substantially all of the surface area of said container to greater than 30 % of the radiation in the range of 240 to 280 nm which impinges upon said container, and wherein said container is impervious to microorganisms.

This invention further provides a container for a contact lens comprising a lidstock wherein said lidstock is transmissive to greater than 30 % of the radiation in the range of 240 to 280 nm directed at said lidstock.

The containers of this invention provide a means for storing medical devices, preferably contact lenses and/or liquids in a sterile environment for a period of time, without requiring the addition of any chemical additives.

### DESCRIPTION OF THE INVENTION

The containers of this invention are particularly useful for housing medical devices while sterilizing the medical device using UV radiation. The UV radiation can be provided to the medical device by any method or apparatus.

Pulsed UV radiation processes and devices are disclosed in WO96/0977, and US Patents 5,768,853; 4,464,336; 5,786,598; 5,034,235 and 4,871,559 incorporated herein by reference. The preferred embodiment involves the sterilization of a contact lens in a contact lens container, using UV radiation which impinges upon the container from substantially all directions.

The medical device container of this invention comprises materials which are transmissive to UV radiation so that UV radiation can penetrate the container and reach all the surfaces of the medical device to be sterilized. The medical device is either transmissive to UV radiation or is such that it creates no shadowing for microorganisms to "hide" from the UV radiation on surfaces where the microorganisms are to be inactivated. Preferably the container is transmissive to UV radiation over substantially the entire surface area of the container. Preferably the container is transmissive to greater than 30 % of the radiation in the range of 240 to 280 nm which impinges upon said container, more preferably the container is transmissive to greater than 40 % of the radiation in the range of 240 to 280 nm which impinges upon said container, and most preferably the container is transmissive to greater than 50 % of the radiation in the range of 240 to 280 nm which impinges upon said container. The percentage of radiation transmission in the range of 240 to 280 may be measured at one or more wavelengths within the range; however, preferably the percentage of transmission of radiation through the container is a total percentage over the entire 240 to 280 nm range. In the preferred embodiments the container is transmissive to the UV radiation at the specified levels over substantially all the surfaces of the container.

The containers can take any form, including bags, tubes, cylinders, bottles, vials, cartons, and shrink-wrap over medical devices. The preferred containers preferably comprise a base and a top. The base can be a flat or formed material, and the top can be a flat or formed material depending upon the medical device to be housed within the container. The only requirement is that the container is impenetrable to microorganisms during the time that the medical device is sterilized using UV radiation, and for the shelf life of the medical device or container, or until the container is opened by the end-user of the device. Alternatively, the container can be impenetrable to microorganisms during the time that the medical device is sterilized using UV radiation and then additional packaging can be added to the container after sterilization to provide a package which is impenetrable to microorganisms for the shelf life of the medical device or the container, or until the container is opened by the end-user of the device.

Useful materials for the container of this invention include polyolefins, such as, polyethylenes, polypropylenes, polybutylenes, and copolymers of the above; cycloolefins (COC); halogenated films, such as polyvinychlorides (PVC), polyvinylidine chlorides (PVDC), polymonochlorotrifluoroethylenes (PCTFE), polyvinylidine fluorides (PVDF), and polyfluorocarbons; polyurethanes; polyamides; polyimides; ethylene-vinyl acetate copolymers (EVA); ethylene vinyl alcohols (EVOH); ethylene acrylic acid copolymers (EAA); acrylics, such as polymethylmethacrylates; ionomers; and cellulose materials, such as cellulose esters, and cellophanes. The more preferred materials are polyolefins, such as polyethylenes, polypropylenes, polybutylenes, cycloolefins, and copolymers of the above, polyamides, and PCTFE.

In the preferred embodiment the container is a contact lens container. In the preferred embodiment the contact lens container has a conventional shape, that is, the base of the container has a recessed area for housing the contact lens, a seal area around the recessed area, and tab for gripping to hold the container. The base of the container for contact lenses is often referred to as the bowl. Preferably, the top of the container is a lid which is sealed to the base. Preferably the lid comprises a flexible lidstock which can be sealed in the seal area to the bowl to provide a container which is impenetrable to microorganisms. The preferred lidstock is typically a thin flexible sheet which is hermetically sealed to the bowl. The preferred lidstock is peelable. The preferred lidstock is heat-sealed to the bowl. The lidstock is transmissive to greater than 30 %, more preferably greater than 40 %, most preferably greater than 50 % of the radiation in the range of 240 to 280 nm which impinges upon it. More preferably the lidstock and the bowl are transmissive to greater than 30 %, more preferably greater than 40 %, most preferably greater than 50 % of the radiation in the range of 240 to 280 nm which impinges upon them.

The contact lens container of this invention preferably comprises a lidstock wherein said lidstock preferably comprises at least one layer of plastic material. The lidstock can comprise a single plastic layer alone, multiple plastic layers, or at least one layer of plastic and other layers of materials which are not plastic. The preferred plastics are thermoplastics. Presently the preferred lidstock is multilayered in which complementary material layers are selected to provide one or more of the following: moisture barrier, sealability, stiffness, microbiological barrier, heat-resistance, and strength.

The container of this invention comprises a multilayered lidstock which comprises at least a sealant layer (closest to the base) and a heat-resistant layer. The sealant layer is dependent on the method of sealing and the composition of the base. Because the method of sealing is heat-sealing, it is preferred that the heat-sealing layer comprises a material with a low melting point over a wide range, and that the heat-sealing material is compatible with the base material. The preferred base materials are described in more detail below, however, the preferred base materials are polyolefins. Therefore, for the preferred embodiments, the sealant layer is preferably a polyolefin, e.g. polyethylene, polypropylene or a copolymer of polyolefins, such as acrylic acid and maleic anhydride copolymers. In the preferred embodiment in which the base is a polypropylene bowl, the preferred heat-sealing material is polypropylene, an olefin copolymer or cycloolefin polymer.

The heat-resistant layer is a layer consisting of a polyamide, preferably biaxally oriented polyamide (OPA-6), or OPA-6,6 or a cellophane, bonded together with an adhesive layer into a three-layer structure.

The lidstock may comprise one or more adhesive layers. Suitable adhesives for the adhesive layer(s) are vinyl chloride copolymers, vinyl chloride-vinyl acetate copolymers, polymerisable polyesters, vinylpyridine polymers, butadiene-acrylonitrile-methacrylic acid copolymers, phenol resins, acrylic resins, acrylic resins with phenol or acrylate polymers, urethane-modified acrylics, polyester-co-polyamides, polyisobutylenes, polyurethanes, ethylene-acrylic acid mixed polymers, and ethylene-vinyl acetate mixed polymers. The preferred adhesives are selected from the group consisting of aliphatic polyesters and polymerisable polyesters. The most preferred adhesives are aliphatic polyisocyanates. In a preferred embodiment, the lidstock comprises three layers of materials, that is, the sealant layer, the heat-resistant layer and an adhesive layer between the sealant and heat-resistant layers.

The lidstock may comprise a moisture barrier layer. The preferred moisture barrier layer materials comprise silicon oxide, PCTFE, cast (CPP) or biaxally oriented polypropylene (BOPP), PVDC, and COC. The silicon oxide layer is preferably deposited in a vacuum as a vapor chemical deposition onto another layer in the lidstock, e.g. a polyolefin or a polyamide layer. Preferably, the biaxally oriented polyolefin is used in combination with a cast polyolefin sealant layer in the lidstock. The moisture barrier layer is preferably added between the sealant and the heat-resistant layers. In a preferred embodiment, the lidstock comprises five layers of materials: a heat-resistant layer, an adhesive layer, a moisture barrier layer, an adhesive layer and a sealant layer.

In embodiments in which blocking the transfer of oxygen through the container is important, an oxygen barrier layer can be provided. Examples of useful materials for such a layer include silicon oxide, polyacrylonitrile (PAN), PVDC, and EVOH. Particularly advantageous as an oxygen barrier layer is the deposition of a silicon oxide layer onto biaxially oriented polyamide films.

Additional layers or thicker layers of materials may be added in any of the above embodiments for whatever characteristics, e.g. increased moisture barrier properties or increased strength, the lidstock requires. For example, for increased strength, either the thickness of the layers may be increased or an additional layer, e.g., polyolefin layer may be added between the layers specified. Note that the materials listed for each layer may provide more than one benefit, e.g., the heat-resistant layer materials may also increase moisture barrier properties, and/or increase stiffness, etc.

A first preferred embodiment of a lidstock of this invention includes a sealant layer comprising a polyolefin, preferably a polybutylene-polyethylene copolymer having a thickness from 5 to 100 microns, preferably from 20 to 75 microns, next to an aliphatic polyester adhesive layer, preferably an aliphatic polyisocyanate having a thickness of from 1 to 10 microns, preferably 1.5 to 5 microns, next to a heat-resistant layer comprising a polyamide, preferably a biaxally oriented polyamide having a thickness of 5 to 50 microns, more preferably 12 to 30 microns.

A second preferred embodiment comprises the same sealant, adhesive, and heat-resistant layers of the first embodiment with a cast or biaxally oriented PCTFE layer as a moisture barrier layer having a thickness from 10 to 100 microns, preferably from 15 to 50 microns, and an additional adhesive layer between the heat-resistant layer and the sealant layer such that the PCTFE layer is between the two adhesive layers. The PCTFE layer also functions as a stiffness layer.

A third preferred embodiment comprises the same sealant and adhesive layers of the first embodiment, and a silicon oxide coated biaxally oriented polypropylene (BOPP) as the heat-resistant layer having a total thickness from 10 to 100 microns, preferably from 15 to 50 microns, whereby the silicon oxide layer is between the BOPP-film and the adhesive. The thickness of the silicon oxide layer is preferably less than 1 micron. The silicon oxide layer is a moisture barrier layer too.

A fourth preferred embodiment comprises the same sealant and adhesive layers of the first embodiment, and a PVDC coated BOPP, whereby the BOPP is the heat-resistant layer having a total thickness from 10 to 100 microns, preferably from 15 to 50 microns, and the PVDC layer is a moisture and oxygen barrier layer. The thickness of the PVDC layer is preferably from 2 to 5 microns. The PVDC layer is between the BOPP and the adhesive layer.

A fifth preferred embodiment comprises the same sealant and adhesive layers and a biaxally-oriented PVDC film having a total thickness from 10 to 75 microns, preferably from 15 to 50 microns as the heat-resistant layer next to the adhesive layer.

Additional preferred embodiments are all five of the preferred embodiments just described modified to include an additional stiffness layer. In the preferred embodiments, at least one cycloolefin, polypropylene or PCTFE layer is added between the sealant layer and the heat-resistant layer. Preferably at least one additional adhesive layer is added adjacent to the added stiffness layer. One preferred location for the stiffness layer is adjacent to the sealant layer. Preferably an additional adhesive layer is added between the sealant layer and the added stiffness layer. If the stiffness layer is a polypropylene layer, the preferred thickness of the polypropylene stiffness layer is from 20 to 200 microns, more preferably from 30 to 75 microns. The preferred thickness of the PCTFE stiffness layer is from 10 to 100 microns, more preferably 15 to 50 microns.

A sixth preferred embodiment of a lidstock of this invention which includes a stiffness layer comprises a polyolefin, preferably a polybutylene-polyethylene copolymer having a thickness from 5 to 100 microns, preferably from 20 to 75 microns as the sealant layer, next to an aliphatic polyester adhesive layer, preferably an aliphatic polyisocyanate having a thickness of from 1 to 10 microns, preferably 1.5 to 5 microns, next to a cast polypropylene stiffness layer having a thickness from 20 to 200 microns, next to a second aliphatic polyester adhesive layer, preferably an aliphatic polyisocyanate having a thickness of from 1 to 10 microns, preferably 1.5 to 5 microns, next to a heat-resistant layer comprising a polyamide, preferably a biaxally oriented polyamide having a thickness of 5 to 50 microns, more preferably 12 to 30 microns.

A seventh preferred embodiment of a lidstock of this invention which includes a stiffness layer comprises a polyolefin, preferably a polybutylene-polyethylene copolymer having a thickness from 5 to 100 microns, preferably from 20 to 75 microns as the sealant layer, next to an aliphatic polyester adhesive layer, preferably an aliphatic polyisocyanate having a thickness of from 1 to 10 microns, preferably 1.5 to 5 microns, next to a cast cycloolefin polymer layer as the stiffness layer having a thickness from 20 to 200 microns, next to a second aliphatic polyester adhesive layer, preferably an aliphatic polyisocyanate having a thickness of from 1 to 10 microns, preferably 1.5 to 5 microns, next to a heat-resistant layer comprising a polyamide, preferably a biaxally oriented polyamide having a thickness of 5 to 50 microns, more preferably 12 to 30 microns. The cycloolefin also acts as a moisture barrier layer.

An eighth preferred embodiment of a lidstock of this invention which includes a stiffness layer comprises a polyolefin, preferably a polybutylene-polyethylene copolymer having a thickness from 5 to 100 microns, preferably from 20 to 75 microns as the sealant layer, next to an aliphatic polyester adhesive layer, preferably an aliphatic polyisocyanate having a thickness of from 1 to 10 microns, preferably 1.5 to 5 microns, next to a silicon oxide coated cast polyolefin layer, such as silicon oxide coated polypropylene stiffness layer having a thickness from 20 to 200 microns, next to a second aliphatic polyester adhesive layer, preferably an aliphatic polyisocyanate having a thickness of from 1 to 10 microns, preferably 1.5 to 5 microns, next to a heat-resistant layer comprising a polyamide, preferably a biaxally oriented polyamide having a thickness of 5 to 50 microns, more preferably 12 to 30 microns. The silicon oxide layer is preferably closer to the sealant layer than the polyolefin layer on which it was deposited. Further, the silicon oxide is a moisture barrier layer too.

Another example of one preferred embodiment of a lidstock of this invention including a stiffness layer comprises a polyolefin, preferably a polybutylene-polyethylene copolymer having a thickness from 5 to 100 microns, preferably from 20 to 75 microns as the sealant layer, next to an aliphatic polyester adhesive layer, preferably an aliphatic polyisocyanate having a thickness of from 1 to 10 microns, preferably 1.5 to 5 microns, next to a biaxially oriented PVDC stiffness layer having a thickness from 10 to 100 microns, preferably 10 to 50 microns, next to a second aliphatic polyester adhesive layer, preferably an aliphatic polyisocyanate having a thickness of from 1 to 10 microns, preferably 1.5 to 5 microns, next to a heat-resistant layer comprising a polyamide, preferably a biaxally oriented polyamide having a thickness of 5 to 50 microns, more preferably 12 to 30 microns. The PVDC layer is a moisture and oxygen barrier layer also.

The preferred total thickness of the lidstock should be from 20 to 300 microns, more preferably from 50 to 150 microns. The water vapor transmission rate through the lidstock and the container should be less than 5 grams per 100 sq. inches per day, more preferably less than 0.1 grams per 100 sq. inches per day, and most preferably less than 0.05 grams per 100 sq. inches per day at ambient conditions 23°C and 50 % RH. Preferably the lidstock, after sealing to the base of the container, provides a peel strength of between 400 and 1400 grams per linear inch, more preferably between 400 and 1000 grams per linear inch when peeled at an angle of 90 degrees on an Instron device.

The multilayered lidstock can be made by adhesive lamination if adhesives are used, or by extrusion lamination of the heated layers of materials which are thereby melt bonded together. Further, adhesion may be generated or enhanced by the use of high energy sources such as electron beam. Further, thin layers may be deposited by vapor deposition. The method of laminating includes the bonding of the layers over the entire area of the layers or alternatively only in specified areas of the layers, e.g. around the perimeter of the layers. For some of the multilayered embodiments the layers are assembled in separate steps which may allow time for curing of the materials as will be seen in the examples below; however with different equipment it is possible to make the multilayered materials in one step and cure the multilayers together. One or more of the surfaces of the layers of the lidstock of this invention can be treated at any time during the formation of the lidstock. Examples of such treatments include corona treatment, plasma treatment, ion implantation, radiation treatment, and chemical treatments. If necessary, the preferred method of treating a surface layer is by corona discharge treatment, and if an adhesive layer is added, it is preferred to corona discharge treat a thermoplastic layer prior to the addition of an adhesive layer to the thermoplastic layer.

Most of the materials described for use in the container of this invention can be made by conventional methods; however, it is preferred that the materials not contain any substantial quantities of additives that will detrimentally impact the materials' UV radiation transmissivity. Additives to avoid include bulk fillers, lubricants, heat stabilizers, clarifiers, nucleating agents, and anti-microbial oxidants. Other additives to avoid include UV-blockers, pigments and fillers added to provide UV stability. Examples of specific materials that are often added to thermoplastics and adhesives and should be avoided in the containers of this invention include components containing aromatic elements, anti-blocking agents, such as glass and calcium carbonate, slip additives, such as stearate based products (calcium stearate, zinc stearate, etc), and rubber anti-tack additives in high concentrations, such as 5 to 10%. The materials used in the container of this invention should be substantially free of these fillers, and additives, meaning that the materials should comprise less than 10 %, more preferably less than 5 % and most preferably less than 3 % of such components. Due to such additives, commercially available materials can vary greatly in the UV radiation they transmit. For example, a polyolefin film, Rayopeel® RS transmitted 1 % as compared to Rayopeel® Super which transmitted 55 % of the radiation at 240 nm. The Rayopeel® materials are available from Amcor/Transpac. Further, a urethane adhesive Tycel® 7900/6800 transmitted 0.1 % as compared to Tycel® 7909/7283 which transmitted 18 % at 240 nm. The Tycel® adhesives are available from Henkel.

The memory of the thermoplastic materials can be predispositioned or oriented as shrink films, stretch films, uniaxial films, biaxial films, unoriented films, and cast films. The surface characteristics of biaxially oriented films is particularly well suited for low diffraction of UV light and maximizes the transmission through the lidstock. The low additive concentration of most biaxially oriented polyolefins and polyamides for instance also enhances UV transmission. The base can comprise glass and thermoplastics. The base preferably comprises a molded thermoplastic, preferably a polyolefin or cycloolefin, most preferably polypropylene or polyethylene or a copolymer of polypropylene and polyethylene or a cycloolefin. These materials are preferred, because they are well-suited to heat-sealing and provide a high UV transmission, combined with adequate moisture barrier properties. Such materials are commercially available and known to a person of ordinary skill in the art; however, the commercially available materials need to be analyzed to assure sufficient transmission of the UV radiation at 254 nm, due to additives, such as fillers, slip additives, anti-blocking agents, etc which may have been added to the composition by the producer of the material. (This was described earlier for the lidstock materials.) For example, polypropylenes from two different manufacturers provided different transmissivities: 0.5mm thick pieces of polypropylene, Exxon 1605 and 1105 provide 50% transmission at 254 nm, whereas a 0.5 mm thick piece of polypropylene Montel Himont 701 was <5-10% transmission at 254 nm. The UV radiation transmission can be measured by using near infrared Spectrophotometry, e.g., Perkins Elmer Lambda 19 equipment. If the transmission is too low, the composition of the bowl material can be modified to remove additives e.g. fillers, and blockers, clarifiers, nucleating agents or a different material will have to be used. Further, the molding process conditions may effect the transmissivity, and can be modified in an effort to increase transmissivity. Finally, the shape or thickness of the base can be modified to increase the transmissivity. Typically a thinner part will have a higher transmissivity as compared to a thicker part. The preferred base is a 0.5 mm thick

This invention is further described and illustrated by the examples, which follow.

### Example 1

The lidstock of this example consisted of the materials listed in Table 1. From the top of the table to the bottom of the table, the materials are the heat-resistant layer, an adhesive layer, a stiffness layer, a second adhesive layer, and a sealant layer. The layers were assembled in two steps. In the first step, cast polypropylene (CPP) was adhesive laminated to oriented polyamide film (oPA) at ambient conditions and cured for 24 hrs. In the second step, the product from the first step was adhesive laminated to the sealant layer which consisted of a low density polyethylene-polybutylene peel film. The lidstock was then cured under ambient conditions for 5 days.

**TABLE 1**

| Material | Thickness (micron) | Weight (g/m²) | Tolerance (g/m²) |
|---|---|---|---|
| OPA, Emblem® 1200 from Allied Signal | 12 | 13.8 | 1.4 |
| Aliphatic Polyisocyanate adhesive system, Tycel® 7992/7294 from Henkel | ---- | 1.8 | 0.5 |
| CPP, Solmed® 200 from Solvay | 120 | 109.2 | 10.9 |
| Aliphatic Polyisocyanate adhesive system, Tycel® 7992/7294 from Henkel | ----- | 1.8 | 0.5 |
| Polyethylene sealant, Rayopeel® Super from Amcor/Transpac | 50 | 46.6 | 4.6 |

This lidstock was successfully heat-sealed to the preferred polypropylene base, Exxon 1105, at 180-205°C using a heat-sealing device. The dwell time in the heat sealer was 0.5 to 5.0 seconds. The force was approximately from 3 to 5 Bar.

Using a Perkin Elmer Lambda 19, the lidstock measured 53 % transmission at 253.7 nm, and the bowl (0.5mm thick) measured 56.9 % transmission at 249.5 nm at the center. The water vapor transmission of the lidstock was less than 0.33 gr./100 sq. inches/day, and the Instron Peel Strength test of the heat-sealed lidstock from the bowl was 400 to 900 grams per linear inch.

### Example 2

The lidstock of this example consisted of the materials listed in Table 2. From the top of the table to the bottom of the table, the materials consisted of a heat-resistant layer, an adhesive layer and a sealant layer. The same materials used for these layers in Example 1 were used in Example 2.

The biaxially oriented polyamide film was adhesive coated and joined to the sealant layer in one lamination step. The lidstock was cured for 5 days.

**TABLE 2**

| Material | Thickness (micron) | Weight (g/m²) | Tolerance (g/m²) |
|---|---|---|---|
| OPA Emblem® 1200 from Allied Signal | 12 | 13.8 | 1.4 |
| Aliphatic Polyisocyanate Adhesive System Tycel® 7992/7294 from Henkel | ---- | 1.8 | 0.5 |
| Polyethylene Rayopeel® Super from Amcor/Transpac | 50 | 46.6 | 4.6 |

This lidstock was successfully heat-sealed to the preferred polypropylene base at 160-190°C. The dwell time in the heat sealer was 0.3 to 3.0 seconds. The force was approximately 1.5 to 4 Bar.

Using a Perkin Elmer Lambda 19, the lidstock measured 62.1 % transmission at 253.7 nm. The water vapor transmission of the lidstock was consistently less than 1.18 grams/100 sq. in./day, and the Instron Peel Strength test of the heat-sealed lidstock from the bowl was between 400 to 900 grams per linear inch.

### Example 3

The lidstock of this example consisted of the materials listed in Table 3. From the top of the table to the bottom of the table, the materials are the heat-resistant layer, an adhesive layer, a moisture barrier layer, a stiffness layer, a second adhesive layer, and a sealant layer. The layers were assembled in two steps. The materials used in this example were the same as those used in Example 1 except for the stiffness layer and the moisture barrier layer. The stiffness layer in this example was a silicon oxide coated BOPP, the silicon oxide was also a moisture barrier layer. The layers were assembled in three steps. In the first step, silicon oxide, Ceramis® by Lawson Mardon Packaging, was vapor deposited in a vacuum on one side of a biaxially oriented polypropylene (BOPP) film. In a second step, the silicon oxide coated BOPP was adhesive laminated to the biaxially oriented polyamide layer, and was cured for twenty-four hours. In a third step, the product of the second step was adhesive laminated to the sealant layer and was cured for five days.

**TABLE 3**

| Material | Thickness (micron) | Weight (g/m²) | Tolerance (g/m²) |
|---|---|---|---|
| OPA Emblem® 1200 from Allied Signal | 12 | 13.8 | 1.38 |
| Aliphatic Polyisocyanate Adhesive System, Tycel® 7992/7294 from Henkel | ---- | 1.8 | 0.5 |
| BOPP, Propafilm® from ICI | 20 | 18.40 | 1.84 |
| Silicon Oxide layer, Ceramis® CO-H-XD from Lawson Mardon | < 0.1 | ---- | ---- |
| Aliphatic Polyisocyanate Adhesive System, Tycel® 7992/7294 from Henkel | ----- | 1.8 | 0.5 |
| Polyethylene sealant, Rayopeel® Super from Amcor/Transpac | 50 | 46.6 | 4.6 |

This lidstock was successfully heat-sealed to the preferred polypropylene base at 170-210°C. The dwell time in the heat sealer was 0.5 to 3.0 seconds. The force was approximately 3 to 5 Bar.

Using a Perkin Elmer Lambda 19, the lidstock measured 45.1 % transmission at 253.7 nm. The water vapor transmission of the lidstock was less than 0.03 grams/ 100 sq. inches/day, and the Instron Peel Strength test of the heat-sealed lidstock from the bowl was between 400 to 900 grams per linear inch.

### Example 4

This lidstock used similar materials to those used to form the lidstock of Example 3; however, the order of the materials was changed. From the top of Table 4 to the bottom of the table, the materials are the heat-resistant layer, an adhesive layer, a stiffness layer, a second adhesive layer, a moisture barrier layer, and a sealant layer. In this example, the silicon oxide layer was coated onto the BOPP sealant layer, unlike Example 3. The lidstock of this example was made in three steps. In the first step, cPP was adhesive laminated to oPA and cured for twenty-four hours. In a second step, silicon oxide was vapor deposited in a vacuum onto one side of the BOPP. In a third step, the products of steps 1 and 2 were adhesive laminated to form the lidstock. The lidstock was then cured for five days.

**TABLE 4**

| Material | Thickness (micron) | Weight (g/m ² ) | Tolerance (g/m ² ) |
|---|---|---|---|
| OPA, Emblem® 1200 from Allied Signal | 12 | 13.8 | 1.38 |
| Aliphatic Polyisocyanate Adhesive System, Tycel® 7992/7294 from Henkel | ----- | 1.8 | 0.5 |
| CPP, Solmed 200 from Solvay | 120 | 109.2 | 10.92 |
| Aliphatic Polyisocyanate Adhesive System, Tycel® 7992/7294 from Henkel | ----- | 1.8 | 0.5 |
| Silicon oxide, Ceramis® CO-C-XD from Lawson Mardon | < 0.1 | ---- | ---- |
| BOPP, Shorco® from Courtaulds | 20 | 18.40 | 1.84 |

This material was successfully heat-sealed to the preferred polypropylene base at 160-190°C. The dwell time in the heat sealer was 1.0 to 5.0 seconds. The force was approximately 1.0 to 5.0 Bar.

Using a Perkin Elmer Lambda 19, the lidstock measured 50.3 % transmission at 253.7 nm. The water vapor transmission of the lidstock was less than 0.06 grams/100 sq. inches/day, and the Instron Peel Strength test of the heat-sealed lidstock from the bowl was between 400 and 900 grams per linear inch.

### Example 5

The materials used to make the lidstock of this example are listed in Table 5. A different biaxially oriented polypropylene sealant layer and a different biaxially oriented polyamide heat-resistant layer were used. The biaxially oriented polypropylene has a coextruded polyethylene-polypropylene copolymer sealant layer to seal and peel from the polypropylene bowl. In the table from top to bottom are a heat-resistant layer, an adhesive layer and a sealant layer. This lidstock was made in a single step by is adhesive laminating the biaxially oriented polypropylene to the biaxially oriented polyamide. The lidstock was then room temperature cured for five days.

**TABLE 5**

| Material | Thickness (micron) | Weight (g/m²) | Tolerance (g/m²) |
|---|---|---|---|
| OPA, LP-5 from Mitsubishi | 15 | 17.7 | 1.80 |
| Aliphatic Polyisocyanate Adhesive System, Tycel® 7992/7294 from Henkel | ----- | 1.8 | 0.5 |
| Biaxially oriented Polypropylene, Rayopp® RGP 100 from UCB | 25 | 22.7 | 2.30 |

This material was successfully heat-sealed to the preferred polypropylene base at 150-175°C. The dwell time in the heat sealer was 0.3 to 1.75 seconds. The force was approximately 0.5 to 3.0 Bar.

Using a Perkin Elmer Lambda 19, the lidstock measured 60.5 % transmission at 253.7 nm. The water vapor transmission of the lidstock was less than 1.0 grams per 100 sq. inches/day, and the Instron Peel Strength test of the heat-sealed lidstock from the base was between 400 and 900 grams per linear inch.

### Example 6

The materials used to make this lidstock are listed in Table 6. The PCTFE layer provides significant moisture barrier properties. The biaxially oriented polypropylene has a coextruded polyethylene-polypropylene copolymer sealant layer to seal and peel from the polypropylene base. In the table from the top are a heat-resistant/moisture barrier layer, an adhesive layer and a sealant layer.

**TABLE 6**

| Material | Thickness (micron) | Weight (g/m²) | Tolerance (g/m²) |
|---|---|---|---|
| PCTFE, Aclar® NT from Allied Signal | 33 | 59.4 | 6.0 |
| Aliphatic Polyisocyanate Adhesive System, Tycel® 7992/7294 from Henkel | ----- | 2.2 | 0.5 |
| Biaxially oriented Polypropylene, Rayopp® RGP 100 from UCB | 25 | 22.7 | 2.30 |

This material was successfully heat-sealed to the preferred polypropylene base at 150-175°C. The dwell time in the heat sealer was 0.3 to 1.5 seconds. The force was approximately 0.5 to 3.0 Bar.

Using a Perkin Elmer Lambda 19, the lidstock measured 71.4 % transmission at 253.7 nm. The water vapor transmission of the lidstock was less than 0.5 grams per 100 sq. inches/day, and the Instron Peel Strength test of the heat-sealed lidstock from the base was between 400 and 900 grams per linear inch.

The examples show that it is possible with the right combination of materials to make a lidstock that is transmissive to UV radiation and still has the necessary characteristics for use as a contact lens container. The description of the preferred embodiments and specific examples can be expanded upon to make other containers to house medical devices which are, for example, to be sterilized using UV radiation. Such containers would be within the scope of the claims below.

## Claims

1. A medical device container, wherein said container is transmissive over substantially all of the surface area of said container to greater than 30% of the radiation in the range of 240 to 280 nm which impinges upon said container, wherein said container is impervious to microorganisms, and wherein at least a portion of said container comprises a multilayer material, a heat sealable first layer of said multilayer material comprising a material selected from the group consisting of polyolefins, polyolefin copolymers, cycloolefin polymers , cycloolefin copolymers, and a heat resistant second layer of said multilayer material comprising a material selected from the group consisting of polyamides and cellophane, wherein said heat sealable first layer is laminated to said heat resistant second layer by an adhesive layer.

2. The medical device container of claim 1 wherein said heat sealable first layer comprises a material selected from the group consisting of polypropylene, polyethylene, co-polymers of polypropylene and co-polymers of polyethylene.

3. The medical device container of claim 1, wherein said adhesive layer comprises a material selected from the group consisting of vinyl choride copolymers, vinyl chloride-vinyl acetate copolymers, polymerisable polyesters, vinylpyridine polymers, butadiene-acrylonitrile-methacrylic acid copolymers, phenol resins, acrylic resins, acrylic resins with phenyl or acrylate polymers, urethane-modified acrylics, polyester-co-polyamides, polyisobutylenes, polyurethanes, ethylene-acrylic acid mixed polymers and ethylene-vinyl acetate mixed polymers.

4. The medical device container of claim 3, wherein said adhesive comprises a material selected from the group consisting of aliphatic polyesters and polymerisable polyesters.

5. The medical device container of claim 1, wherein said container is transmissive over substantially all of the surface area of said container to greater than 40% of the radiation in the range of 240 to 280 nm which impinges upon said container.

6. The medical device container of claim 1, wherein said heat sealable first layer is a polyolefin layer having a thickness from 5 to 100 microns, and said heat resistant second layer comprises a polyamide layer having a thickness from 5 to 50 microns.

7. The medical device container of claim 1, wherein said heat resistant second layer is biaxally oriented polyamide.

8. The medical device container of claim 1, wherein said multilayer material contains less than 10% bulk fillers, lubricants, heat stabilizers, clarifiers, nucleating agents, anti-microbial oxidants, UV-blockers, pigments and fillers added to provide UV stability.

9. The medical device container of claim 1, wherein said container is less than 10% of components containing aromatic elements, anti-blocking agents, glass, calcium carbonate, slip additives, stearates, and rubber anti-tack additives.

10. The medical device container of claim 1, wherein said container is a contact lens container comprising a base and a top, and wherein said top comprises said multilayer material.

11. The medical device container of claim 1, wherein said top is a lidstock and said lidstock is peelable from said base.

12. The medical device container of claim 1 wherein said heat sealable first layer comprises biaxially oriented polypropylene and said heat resistant second layer comprises biaxally oriented polyamide film.

13. The medical device container of claim 1 wherein said heat sealable first layer comprises polyethylene-polybutylene peel film and said heat resistant second layer comprises oriented polyamide film.

## Patentansprüche

1. Behälter für ein Medizinprodukt, wobei der Behälter im wesentlichen über den gesamten Oberflächenbereich des Behälters für mehr als 30% der Strahlung im Bereich von 240 bis 280 nm, die auf den Behälter trifft, durchlässig ist, wobei der Behälter für Mikroorganismen undurchlässig ist und wobei mindestens ein Teil des Behälters ein vielschichtiges Material, eine durch Hitze versiegelbare erste Schicht des vielschichtigen Materials, umfassend ein Material, ausgewählt aus der Gruppe bestehend aus Polyolefinen, Polyolefin-Copolymere, Cycloolefin-Polymere, Cycloolefin-Copolymere und eine hitzestabile zweite Schicht des vielschichtigen Materials, umfassend ein Material, ausgewählt aus der Gruppe bestehend aus Polyamiden und Cellophan, umfaßt, wobei die durch Hitze versiegelbare erste Schicht auf die hitzestabile zweite Schicht durch eine adhäsive Schicht auflaminiert ist.

2. Behälter für ein Medizinprodukt nach Anspruch 1, wobei die durch Hitze versiegelbare erste Schicht ein Material umfaßt, ausgewählt aus der Gruppe bestehend aus Polypropylen, Polyethylen, Copolymere von Polypropylen und Copolymere von Polyethylen.

3. Behälter für ein Medizinprodukt nach Anspruch 1, wobei die adhäsive Schicht ein Material umfaßt, ausgewählt aus der Gruppe bestehend aus Vinylchlorid-Copolymeren, Vinylchlorid-Vinylacetat-Copolymeren, polymerisierbaren Polyestern, Vinylpyridin-Polymeren, Butadienacrylonitrilmethacrylsäure-Copolymeren, Phenolharzen, Acrylharzen, Acrylharzen mit Phenyl- oder Acrylat-Polymeren, Urethanmodifizierten Acrylen, Polyester-Copolyamiden, Polyisobutylenen, Polyurethanen, Ethylenacrylsäure-gemischten Polymeren und Ethylen-Vinylacetat-gemischten Polymeren.

4. Behälter für ein Medizinprodukt nach Anspruch 3, wobei das adhäsive Mittel ein Material umfaßt, ausgewählt aus der Gruppe bestehend aus aliphatischen Polyestern und polymerisierbaren Polyestern.

5. Behälter für ein Medizinprodukt nach Anspruch 1, wobei der Behälter im wesentlichen über den gesamten Oberflächenbereich des Behälters für mehr als 40% der Strahlung im Bereich von 240 bis 280 nm, die auf den Behälter trifft, durchlässig ist.

6. Behälter für ein Medizinprodukt nach Anspruch 1, wobei die durch Hitze versiegelbare erste Schicht eine Polyolefinschicht ist, die eine Dicke von 5 bis 100 Mikron aufweist und die hitzestabile zweite Schicht eine Polyamidschicht umfaßt, die eine Dicke von 5 bis 50 Mikron aufweist.

7. Behälter für ein Medizinprodukt nach Anspruch 1, wobei die hitzestabile zweite Schicht ein biaxial-orientiertes Polyamid ist.

8. Behälter für ein Medizinprodukt nach Anspruch 1, wobei das vielschichtige Material weniger als 10% Bulkfüllmaterialien, Feuchthaltemittel, Hitzestabilisatoren, Klärmittel, Nukleierungsmittel, anti-mikrobielle Oxidationsmittel, UV-Blocker, Pigmente und Füllmaterialien, hinzugefügt, um eine UV-Stabilität zur Verfügung zu stellen, enthält.

9. Behälter für ein Medizinprodukt nach Anspruch 1, wobei weniger als 10% der Verbindungen des Behälters aromatische Bestandteile, anti-blockierende Agenzien, Glas, Calciumcarbonat, Gleitmittel, Stearate und Additive gegen die Haftfähigkeit von Gummi enthält.

10. Behälter für ein Medizinprodukt nach Anspruch 1, wobei der Behälter ein Kontaktlinsenbehälter ist, der ein Unterteil und ein Oberteil umfaßt und wobei das Oberteil das vielschichtige Material umfaßt.

11. Behälter für ein Medizinprodukt nach Anspruch 1, wobei das Oberteil ein Foliendeckel ist und dieser Foliendeckel vom Unterteil abziehbar ist.

12. Behälter für ein Medizinprodukt nach Anspruch 1, wobei die durch Hitze versiegelbare erste Schicht ein biaxial-orientiertes Polypropylen umfaßt und die hitzestabile zweite Schicht eine biaxial-orientierte Polyamidschicht umfaßt.

13. Behälter für ein Medizinprodukt nach Anspruch 1, wobei die durch Hitze versiegelbare erste Schicht eine abziebare Polyethylen-Polybutylenschicht umfaßt und die hitzestabile zweite Schicht eine orientierte Polyamidschicht umfaßt.

## Revendications

1. Contenant pour un dispositif médical, dans lequel ledit transmet sur sensiblement toute la zone de surface dudit contenant plus de 30 % du rayonnement dans le domaine allant de 240 à 280 nm qui vient heurter ledit contenant, dans lequel ledit contenant est imperméable aux microorganismes, et dans lequel au moins une partie dudit contenant comprend un matériau multicouche, une première couche thermoscellable dudit matériau multicouche comprenant un matériau sélectionné dans le groupe composé de polyoléfines, de copolymères de polyoléfines, de polymères de cyclooléfines, de copolymères de cyclooléfines et une seconde couche thermorésistante dudit matériau multicouche comprenant un matériau sélectionné dans le groupe composé de polyamides et de cellophane, dans lequel ladite première couche thermoscellable est laminée sur ladite seconde couche thermorésistante par une couche adhésive.

2. Contenant pour un dispositif médical selon la revendication 1, dans lequel ladite première couche thermoscellable comprend un matériau sélectionné dans le groupe constitué par le polypropylène, le polyéthylène, les copolymères du polypropylène et les copolymères du polyéthylène.

3. Contenant pour un dispositif médical selon la revendication 1, dans lequel ladite couche adhésive comprend un matériau sélectionné dans le groupe constitué par les copolymères du chlorure de vinyle, les copolymères du chlorure de vinyle-acétate de vinyle, les polyesters polymérisables, de polymères de la vinylpyridine, les copolymères du butadiène-acrylonitrile-acide méthacrylique, les résines phénoliques, les résines acryliques, les résines acryliques avec du phényle ou de polymères d'acrylate, les acryliques modifiés avec de l'uréthane, les polyestercopolyamides, les polyisobutylènes, les polyuréthanes, les polymères mixtes d'éthylène-acide acrylique et les polymères mixtes d'éthylène-acétate de vinyle.

4. Contenant pour un dispositif médical selon la revendication 3, dans lequel ledit adhésif comprend un matériau sélectionné dans le groupe composé de polyesters aliphatiques et de polyesters polymérisables.

5. Contenant pour un dispositif médical selon la revendication 1, dans lequel ledit contenant transmet sur sensiblement toute la zone de surface dudit contenant plus de 40 % du rayonnement allant de 240 à 280 nm qui vient heurter ledit contenant.

6. Contenant pour un dispositif médical selon la revendication 1, dans lequel ladite première couche thermoscellable est une couche de polyoléfine ayant une épaisseur allant de 5 à 100 microns, et ladite seconde couche thermorésistante comprend une couche de polyamide ayant une épaisseur allant de 5 à 50 microns.

7. Contenant pour un dispositif médical selon la revendication 1, dans lequel ladite seconde couche thermorésistante est du polyamide biaxialement orienté.

8. Contenant pour un dispositif médical selon la revendication 1, dans lequel ledit matériau multicouche contient moins de 10 % de fibres en vrac, de lubrifiants, de stabilisants thermiques, de clarifiants, d'agents de nucléation, d'oxydants antimicrobiens, de bloqueurs d'UV, de pigments et de charges ajoutés pour fournir une stabilité aux UV.

9. Contenant pour un dispositif médical selon la revendication 1, dans lequel ledit contenant contient moins de 10 % de composants contenant des éléments aromatiques, des agents antiadhésifs, du verre, du carbonate de calcium, des lubrifiants, des stéarates et des additifs antipégosité pour caoutchouc.

10. Contenant pour un dispositif médical selon la revendication 1, dans lequel ledit contenant est un contenant pour lentilles de contact comprenant une base et un sommet, et dans lequel ledit sommet comprend ledit matériau multicouche.

11. Contenant pour un dispositif médical selon la revendication 1, dans lequel ledit sommet est un opercule et ledit opercule est détachable à partir de ladite base.

12. Contenant pour un dispositif médical selon la revendication 1, dans lequel ladite première couche thermoscellable comprend du polypropylène biaxialement orienté et ladite seconde couche thermorésistante comprend un film de polyamide biaxialement orienté.

13. Contenant pour un dispositif médical selon la revendication 1, dans lequel ladite première couche thermoscellable comprend un film détachable en polyéthylène-polybutylène et ladite seconde couche thermorésistante comprend un film de polyamide orienté.
